# EUROPEAN PATENT SPECIFICATION

(11) **EP 0 769 910 B1**
(45) Date of publication and mention of the grant of the patent: **06.09.2000**
(21) Application number: 95920089.0
(22) Date of filing: 23.05.1995
(51) Int. Cl.: A01N 65/00, B27K 3/34, B27K 3/50, A23L 3/3472, A23J 1/12, A61K 35/78

(54) **PROCEDURE FOR ISOLATING A MICROBE INHIBITOR, A MICROBE INHIBITOR, AND USE OF A MICROBE INHIBITOR**
VERFAHREN ZUR ISOLIERUNG EINES MIKROBENINHIBITORS, MIKROBENINHIBITOR UND SEINE VERWENDUNG
PROCEDE D'ISOLEMENT D'UN INHIBITEUR DE MICROBE, INHIBITEUR DE MICROBE, ET SON UTILISATION

(30) Priority: 08.07.1994 FI 943281
(43) Date of publication of application: 02.05.1997
(73) Proprietor: ALKO-YHTIOT OY, 00180 Helsinki (FI)
(72) Inventor: SAARELAINEN, Ritva, FIN-00200 Helsinki (FI); LÄHDESMÄKI, Matti, FIN-60320 Seinäjoki (FI); SUORANTA, Kari, FIN-02210 Espoo (FI)
(74) Representative: Papula, Antti
(86) International application number: FI9500281
(87) International publication number: WO9601564

(56) References cited:
- EP-A- 0 058 133
- EP-A- 0 542 398
- EP-A- 0 609 780
- SE-B- 457 048
- US-A- 4 976 960

## Description

The present invention concerns a procedure for isolating from cereal a microbe inhibitor and/or a combination of microbe inhibitors. The invention further concerns a microbe inhibitor and/or a combination of microbe inhibitors. Moreover, the invention concerns the use of a microbe inhibitor and/or a combination of microbe inhibitors.

Microbes are in this context in particular understood to be bacteria and various fungi, such as yeast fungi, moulds, decay fungi, trichophytid fungi, etc., and in general all microbes known in themselves which cause damage in economic activity and in health care.

Microbial growth and contamination cause major damage in various fields of industry, business and production. Bacteria mostly cause damage in foodstuff industry, particularly in meat processing industry, and through contamination generally in health care. Yeast and mould fungi cause damage particularly in foodstuff industry, notably in bakery industry, dairy industry, jam and sweets industry, and in production of prepared foods, in fodder industry, timber and board industry, paper and pulp industry, etc. Decay fungi cause damage particularly in wood conversion industry and in paper and pulp industry.

Attempts have been made to fight microbes by greatly different procedures and methods, depending on the branch of industry and kind of product concerned. It has been common practice to apply in microbe fighting e.g. methods of treatment which destroy microbes, such as heat treatment, various irradiation methods, particular chemicals which destroy microbes, etc. Attempts to fight microbes have further been made with the aid of general sanitation, i.e., endeavours have been made to keep the product apart from the microbes responsible for contamination with the aid of protective packaging, protective gas, cleaning or any other procedure. Furthermore, one has endeavoured to inhibit the growth of harmful microbes by means of specific microbe inhibitors, particularly chemicals and microbe inhibitors isolated from nature or from artificial substrates.

In general, the chemicals employed in destroying microbes and/or inhibiting their growth are comparatively dangerous, i.e., the chemicals are in most instances toxic or even dangerous to the environment and to persons coming into contact with them; in this connection may for instance be mentioned chemicals used in timber treatment and the serious environmental problems which they cause. In general, there is at present a need to find and develop new agents inhibiting microbial growth, microbe inhibitors which could be used particularly in foodstuff industry in such manner that they would cause lower toxicity than before to foodstuffs, and in other fields as well.

The object of the present invention is to eliminate the drawbacks mentioned in the foregoing. Specifically, the object of the invention is to disclose a novel microbe inhibitor which is derived from natural substances and which is usable, better than before, particularly in connection with foodstuffs and with other products and fields as well. The object of the invention is specifically to disclose a microbe inhibitor and a combination of microbe inhibitors of lowest possible toxicity, causing the least possible side effects and on the whole superior to those of prior art in its application and other properties, and better fit to use, suitable to be used in a wide range of various fields and various products, particularly in connection with foodstuffs.

Furthermore, the present invention is advantageous as to the cost involved, thereby differing from any other inhibitor produced e.g. by fermenting. Price is nowadays one of the salient factors restricting the use of inhibitors.

EP-A-542398 discloses antioxidant compositions derived from rice, and which are used to conserve foodstuffs. The molecular weight of these agents is not disclosed.

The invention is based on extensive cereal studies that have been carried out and from which unexpectedly the fact has emerged that when barley meal is suspended in water and from the water a microbe inhibitor fraction is separated which has molecular weight less than 30,000, suitably 7,000 to 30,000, advantageously 10,000 to 25,000, a microbe inhibitor fraction inhibiting microbial growth in versatile and efficient manner is obtained which has low toxicity and which is appropriate to be used in the greatest diversity of fields.

In the procedure of the invention the inherent defensive system of plants is utilized. It is known in the art that in plants there occur compounds controlling microbial growth, microbe inhibitors. The plants are using these microbe inhibitors in order to fight plant diseases or other pests which threaten them. The novelty in the invention is particularly that microbe inhibitors have now for the first time ever been observed in aqueous suspensions made from barley meal, that is, being present dissolved in water. The invention is based on this observation and on isolation and separation of the microbe inhibitors from said process waters, employing separation procedures mainly known in themselves in the art.

The microbe inhibitors may be used as they are, e.g. in the form of the aqueous suspensions obtained, e.g. in the bakery industry by admixing the aqueous suspension to the dough, usually in the form of products which are dispensed in liquid form or sprayed, in the form of a product to be admixed in solid form (prepared e.g. by means of inspissation) to the desired material, e.g. to a foodstuff or to another material, or admixed in the form of any further processed product or mixture whatsoever, such as a carrier substance.

In the procedure of the invention the barley is ground by any grinding method known in itself in the art, e.g. dry milling or wet milling, described for instance in the patent application EP 267637. Further, the meal thus obtained is suspended in water e.g. in proportion 1:10 to 10:1, advantageously 2:1 to 5:1. The suspension thus obtained may be treated with an enzyme, e.g. cellulase, as disclosed in the cited, earlier application print, e.g. in association with the conventional starch process. From the solution are separated the starch and fibrous substances by any process known in itself in the art, e.g. by straining, filtration, centrifuging and/or any other separating method known in the art. The microbe inhibitor fraction is separated from the solution thus obtained by centrifuging, ultrafiltration, gel filtration, evaporating and/or any other separating method known in the art.

The microbe inhibitor fraction may be concentrated by conventional methods, e.g. ultracentrifuging, ultrafiltration, gel filtration and/or evaporation, or in the way described in the embodiment examples following below. The microbe inhibitor fraction may be concentrated and dried e.g. to powder form, and it may then also present itself e.g. as a lyophilized, finely divided powder. It may equally be bound to a carrier, e.g. to starch or equivalent. Furthermore, the microbe inhibitor fraction may present itself as a concentrated, free-flowing liquid (e.g. 0 to lOfold or even higher concentration), dry matter content e.g. 0.1 to 50 to 100%. In the concentration process the microbe inhibitor fraction may for instance be ultrafiltered and, if desired, evaporated in vacuum (temperature below 45°C).

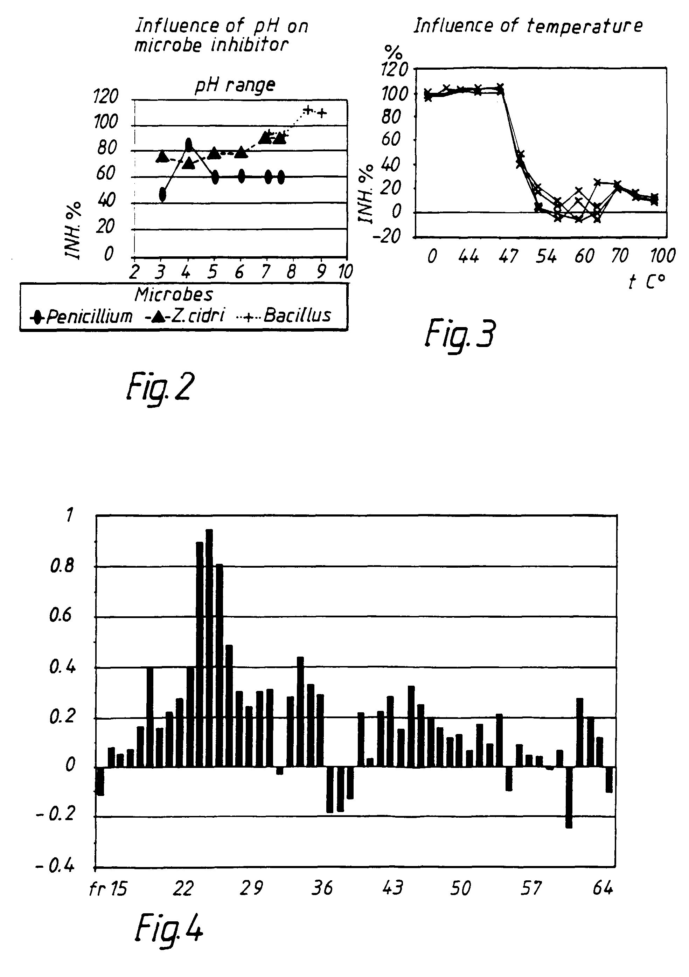

The microbe inhibitor fraction of the invention has been found to tolerate well temperatures up to 50°C; the temperature dependence is shown in Fig. 3; the inhibitor fraction tolerates several hours at temperatures below 50°C (tried up to 5 hrs). The inhibition acticity of the microbe inhibitor measured using Trichoderma as a test organism was after 3 months at room temperature slightly lowered (to get 100 % capacity, one needed 30 % more inhibitor), in refrigerator temperature the activity remained unchanged.

The microbe inhibitor fraction may be further fractionated, e.g. by molecular weight or specific activity, employing procedures known in themselves from molecular fractionating, e.g. ultracentrifuging, ultrafiltration, gel filtration, etc.

The separated and fractionated microbe inhibitors may be used as fractions, i.e., as microbe inhibitors having certain given activities, or as a combination constituted by a plurality of inhibitors, in accordance with the specific use and other requirements, e.g. as a single fraction to inhibit a single microorganism or as a combination which has been found to be the most efficient for retarding or inhibiting the growth of bacteria and fungi in general.

In the foodstuff industry, the microbe inhibitor and/or combination of microbe inhibitors produced as taught by the invention may in particular be used to prevent spoiling of bakery products, to prevent spoiling of meat and meat products, prepared food products and/or fast food, to prevent spoiling of jams, berry and food products and/or sweets, and/or to prevent spoiling of products of the dairy industry. Furthermore, the microbe inhibitor and/or combination of microbe inhibitors prepared as taught by the invention is appropriate to be used to prevent spoiling of various salads and dressings. The microbe inhibitor and/or combination of microbe inhibitors according to the invention can also be used together with any known inhibitor, for example with nicine or some chemical inhibitor such as sorbic acid.

When used in connection with foodstuffs, the microbe inhibitor fraction of the invention has been found to prolong the storage, shop and consumption times of foodstuffs, to retard the spoiling of finished foods and to retard mould infestation of bread. Moreover, the microbe inhibitor fraction improves the structure of the bread by increasing the formation of carbon dioxide in the dough about 10 to 20%; it may be dispensed into the bread dough because it will not inhibit baking yeast, as for instance sorbic acid does. In addition, the microbe inhibitor fraction functions also at neutral pH, pH adjustment is not necessary. The microbe inhibitor fraction can be processed in such a way that it gives grain flavour or colour to the product or that no extra flavour or colour is found. Additionally, the acidity of the inhibitor fraction can be adjusted appropriate for the field of application, without any changes in inhibition capacity (tested by a standard method, at pH-range 3.5 - 5.0) The microbe inhibitor fraction of the invention used in connection with foodstuffs may replace some of the presently used chemical preservative agents, such as sorbic acid, propionates and benzoates.

In the paper, pulp and wood conversion industry, the microbe inhibitor and/or combination of microbe inhibitors prepared as taught by the invention is appropriate to be used to prevent spoiling of paper stock, particularly to prevent slime forming and to eliminate process problems caused by slime as well as quality defects of the paper. In the sawmill industry, the microbe inhibitor and/or combination of microbe inhibitors prepared as taught by the invention can be used generally to prevent decay of timber, e.g. to counteract brown rot fungi, white rot fungi and/or soft rot fungi, and to prevent likewise mould attack of timber and/or mould-induced discoloration. In the board industry and elsewhere in the wood conversion industry and in connection with handling of timber raw material, the microbe inhibitor and/or combination of microbe inhibitors prepared as taught by the invention can likewise be used to counteract decay and/or mould attack of timber and defects caused by them.

In the fodder industry, the microbe inhibitor and/or combination of microbe inhibitors prepared as taught by the invention is appropriate for use to counteract spoiling induced by fungi or bacteria and/or mould attack of fodder raw materials and/or finished fodders.

In the field of health care, the microbe inhibitor and/or combination of microbe inhibitors prepared as taught by the invention is appropriate to be used to fight. in general, particularly fungi, such as trichophytid fungi and bacteria, and fungal and bacterial contaminations.

The invention is described in detail in the following with the aid of embodiment examples, referring to the attached drawings, wherein
Fig. 1 presents an industrial procedure according to the invention for preparation of microbe inhibitors from barley, in diagram form,
Fig. 2 illustrates the effect of pH on the inhibition capacity elicited by a microbe inhibitor fraction produced by the procedure depicted in Fig. 1,
Fig. 3 illustrates the effect of temperature on the inhibition elicited by the same fraction,
Fig. 4 presents the degree of inhibition elicited by gel-filtrated microbe inhibitor fractions.

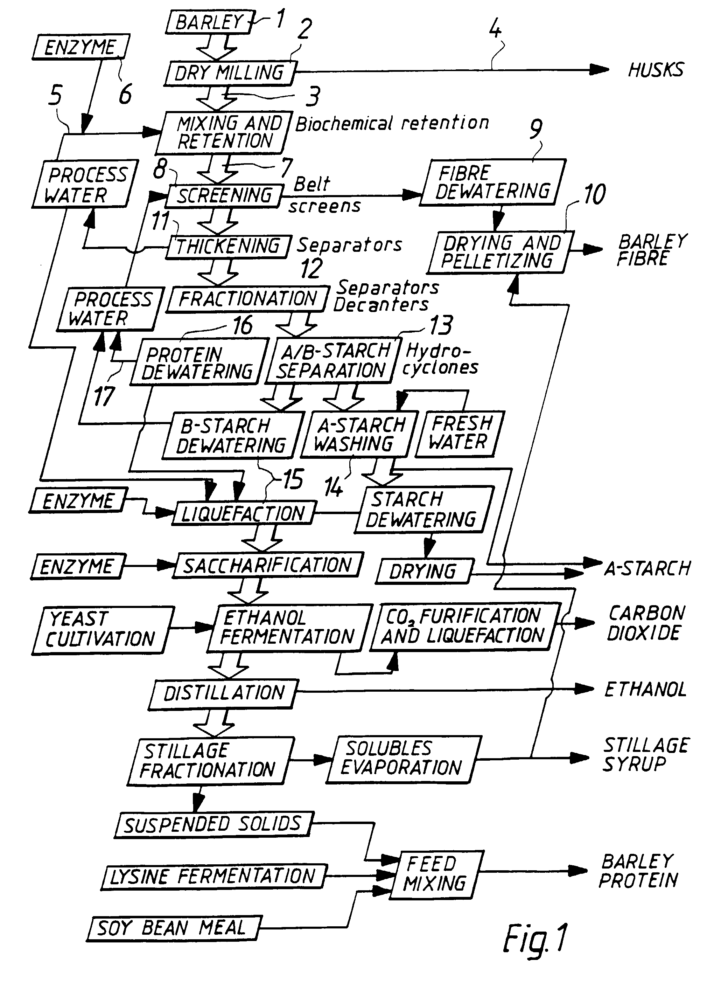

In Fig. 1, the barley is supplied into a hammer mill 2 and ground to meal 3. The husks 4 of the barley are removed from the hammer mill. The meal 3 is suspended in water 5 and thereto is admixed enzyme 6, the suspension 7 thus obtained is strained through a sieve 8. From the coarser fibre fraction thus obtained the water is removed in a dewatering step 9, and the product thus obtained is dried and pelleted in a pelleting step 10. The finely divided suspension from the sieve 8 is conducted to a separation concentrator 11, from which the process water 5 is returned to circulation and the concentrated matter is conducted to separation-fractionation 12; the more concentrated matter is conducted from here to a starch separating process 13,14,15, and the supernatant is conducted to the microbe inhibitor separating step 16. Generally, the microbe inhibitor fraction consists of a water-soluble protein fraction which travels generally e.g. in the above-described starch process along with the process waters. Endeavours are in the separating process to separate the microbe inhibitor fraction from said fraction, using separating methods known in themselves in the art, e.g. methods based on molecular weight.

The microbe inhibitor fraction, molecular weight 7,000 to 30,000, advantageously 10,000 to 25,000, is separated e.g. by the aid of ultracentifuging, ultrafiltration, gel filtration and/or any other separating procedure known in itself in the art. The microbe inhibitor fraction may be further fractionated e.g. on the basis of molecular weight, using ultra centrifuging, ultrafiltration, gel filtration and/or any other separating method known in itself in the art. If desired, the microbe inhibitor and/or combination of microbe inhibitors may be further concentrated, using ultracentifuging, ultrafiltration, gel filtration and/or any other concentrating procedure known in itself in the art.

In one of the research applications of the process according to the figure 1, the microbe inhibitor was prepared by using separation, evaporation and sterile filtration.

Lyophilized microbe inhibitor was also prepared by using process according to the figure 1. The supernatant was centrifuged, sterile filtrated and lyophilized. The lyophilized microbe inhibitor was suspended in water to its original volume and the inhibition activity was measured using the standardmethod. The inhibition activity of the lyophilized inhibitor was the same as the supernatant's.

The effect of pH and temperature on the activity of the microbe inhibitor prepared by the process according to figure 1 was tested.

The effect of pH on the inhibition was tested at pH-range 3-9 using standard method in Trizmabase/HCl or Tris HCl/KOH buffers. The possible growth retardation caused by pH or buffer was taken into consideration. Test organisms were Penicillium C3 bread mold, Zygosaccharomyces cidri and Bacillus stearothermophilus. The results are shown in figure 2.

The effect of temperature on microbe inhibitor capacity was tested. The inhibition fraction was kept 1 to 5 hours at the test temperatures before the measurement of inhibition capacity. The results are shown in figure 3.

To measure the inhibition caused by the microbe inhibitor, a standard method presented in the example 1, was used. Other possible methods to be used are the so-called cup plate assay and continuous growth observation spectrophotometrically by Bioscreen -device (Labsystems, Helsinki, Finland)

### EXAMPLE 1, standard method for measuring the inhibition

The appropriate medium and microbe culture, 150 µl altogether, and inhibitor and water, to the final volume of 165 µl, are added to the microtiter plates. Instead of inhibitor, there is water in the control wells. Three parallel tests of each sample are made. The growth is observed spectrophotometrically at appropriate intervals at 690 nm by Biomeck 1000 (Beckman Instruments Inc. Fullerton, USA). The concentration of the microbe culture (mold spores or bacteria/ yeast) is so chosen that the absorbance in the beginning of the measurement is 0.02 - 0.3. The inhibition rate is counted by comparing the result with the control test. The incubation temperature is the growth temperature of the chosen micro-organism. In the examples presenting the properties of microbe inhibitor, Trichoderma reesei mold has been used as a standard microorganism unless otherwise stated.

The microbe inhibitor fraction prepared as taught by the invention was in tests found to inhibit the following microbes (+++ strong inhibition, ++ distinct inhibition, + weak inhibition, - no inhibition, that is less than 20%):

### Microbes spoiling bakery products

- Yeasts

| | |
|---|---|
| Zygosaccharomyces bailii | ++ |

- Moulds

| | |
|---|---|
| Aspergillus niger | +(++) |
| Cladosporium | ++ |
| Trichosporon | ++ |
| | |
| Bread mould C Penicillium sp | +++ |
| Bread mould 1 Penicillium sp | ++ |
| Bread mould 2 Penicillium sp | +/- |
| Bread mould 4B Rhizopus sp | + |

### Microbes spoiling meat products

- Bacteria

| | |
|---|---|
| Yersinia enterocolitica | +++ |
| Salmonella (Gram, facult. anaer.) enteritidis | ++ |
| Escherichia coli | +/- |
| Bacillus | |
| cereus | ++ |
| thermophilus | +++ |

- Yeasts

| | |
|---|---|
| Debaryomyces hansenii | ++ |
| Rhodotorula glutinis | +++ |
| Trichosporon cutaneum | ++ |

- Moulds

| | |
|---|---|
| Alternaria | ++ |
| Aspergillus niger | ++ |
| Cladosporium | ++ |
| Eurotium | ++ |
| Rhizopus | ++ |

### Microbes spoiling paper pulp stock

| | |
|---|---|
| Alternaria spp. | ++ |
| Cladosporium spp. | ++ |
| Trichoderma spp. | +++ |
| Aspergillus niger | + |
| Penicillium cyclopium | ++ |

### Microbes causing decay of timber

- Brown rots

| | |
|---|---|
| Antrodia sp, Poria sp | +++ |
| Piptoporus betulinus | ++ |

- Dry rot

| | |
|---|---|
| Merulius | +++ |

- Soft rots

| | |
|---|---|
| Trichoderma | + |
| Alternaria | ++ |

### Microbes causing mould attack on timber

| | |
|---|---|
| Cladosporium | ++ |

Generally, the microbe inhibitor fraction was found to inhibit strongly or distinctly the following microbes:

| | |
|---|---|
| Merulius tremellous | +++ |
| Trichoderma reesei | +++ |
| Zygosaccharomyces cidri | +++ |
| Rhodotorula glutinis | +++ |
| Poria placenta | +++ |
| Trichophyton mentagrophytes | +++ |
| Zygosaccharomyces rouxii | ++ |
| Z. bailii | ++ |
| Z. microellips. | ++ |
| Saccharomycodes ludwigii | ++ |
| Trichosporon cutaneum | ++ |
| Eurotium herbariorum | ++ |
| Trichothecium roseum | ++ |
| Cladosporium resinae | ++ |
| Aspergillus awamori | ++ |
| A. ficuum | ++ |
| A. kawachii | ++ |
| A. oryzae | ++ |
| Rhizopus oryzae | ++ |
| Piptoporus betulinus | ++ |
| Acinetobacter | ++ |
| Staphylococcus aureus | +++ |
| Fusarium culmorum | ++ |
| F. graminearum | ++ |
| Leuconostoc mesenteroides | ++ |
| Pseudomonas putida | ++ |

### EXAMPLE 2, gel filtration of the inhibitor fraction

In the present study, a 13 ml sample was taken from the supernatant of the protein separating process in the process of Fig. 1, the sample being diluted and gel-filtrated using Shepadex G 75. In the gel filtration 70 fractions were collected, which were subjected to inhibition testing. Compared to the standard proteins (bovine serum albumine and cytochrome C) the fractions with more than 80 % inhibition capacity are within the range of 10000 to 25000 daltons. The results are presented in Fig. 4 (inhibition 1 = 100%).

### EXAMPLE 3, breadmaking

In the present study a dough was made, mixing proportions for 80 g dough 49 g flour, 30 ml water/salt solution (24 g/l), 0.9 g yeast. The inhibitor was added so that the water quantity decreased correspondingly (the inhibitor had dry matter content 5 to 7%), 8 ml of inhibitor solution were added, better results were achieved with a quantity of 20 ml. The inhibitor fraction was taken from a process as depicted in Fig. 1, supernatant 17, it was centrifuged, the supernatant formed and dialyzed for one day against water.

The dough was made up and baked and the bread was cooled down. The cooled bread was cut into slices, the slices were sprayed with a bread mould mixture and enclosed in bags (Minigrip). The mould attack on the bread slices was followed for about 8 days.

The results are shown in table 1.

**TABLE 1,**

| inhibition of bread moulding | |
|---|---|
| Treatment | Result of comparison (organoleptic) |
| Inh. of inv. | Good |
| Control | Moulded |
| Sorbate | Fair |
| Propionate (16 mg/80 g) | Fair, unpleasent flavour |
| Table 1, inhibition of bread moulding | |

When a microbe inhibitor according to the invention was used, the structure of the bread turned out better in the baking test, owing to the ample carbon dioxide that was formed.

The embodiment examples are intended to illustrate the invention, without delimiting it in any way whatsoever.

## Claims

1. Procedure for separating from cereal a microbe inhibitor and/or combination of microbe inhibitors, **characterized** in that barley is ground, the meal suspended in water in order to dissolve the soluble substances, the insoluble constituents are removed and from the solution is separated a microbe inhibitor fraction having molecular weight 7000 to 30,000.

2. Procedure according to claim 1, **characterized** in that from the solution is separated a microbe inhibitor fraction having molecular weight 10,000 to 25,000.

3. Procedure according to claim 2, **characterized** in that the inhibitor fraction is concentrated, advantageously with the aid of centrifuging, ultrafiltration, gel filtration and/or evaporation.

4. Procedure according to any one of claims 1-3, **characterized** in that from the aqueous suspension of barley meal are separated the starch substances and fibrous substances by the aid of straining, filtration, centrifuging or another known separating method and from the solution thus obtained the microbe inhibitor fraction is separated by centrifuging, ultrafiltration, gel filtration, evaporation or another known separating method.

5. A microbe inhibitor and/or microbe inhibitor fraction which has been separated from cereal, **characterized** in that barley has been ground, the meal has been suspended in water for dissolving the soluble substances, the non-soluble constituents have been removed and from the solution has been separated a microbe inhibitor fraction having molecular weight 7000 to 30,000.

6. A microbe inhibitor and/or microbe inhibitor fraction according to claim 5, characterized in that from the solution has been separated a microbe inhibitor fraction having molecular weight 10,000 to 25,000.

7. A microbe inhibitor and/or microbe inhibitor fraction according to claim 5 or 6, characterized in that the microbe inhibitor fraction has been concentrated, advantageously with the aid of centrifuging, ultrafiltration, gel filtration and/or evaporation.

8. A microbe inhibitor and/or microbe inhibitor fraction according to any of the claims 5-7, **characterized** in that microbe inhibitor fraction has been prepared by separating from the aqueous suspension of barley meal the starch substances and fibrous substances with the aid of straining, filtration, centrifuging or another known separating method and from the solution thus obtained the microbe inhibitor has been separated by centrifuging, ultrafiltration, gel filtration, evaporation or another known separating method.

9. The use of a microbe inhibitor and/or microbe inhibitor fraction as such and optionally with any known inhibitor for retarding or preventing growth of bacteria and/or fungi, for protecting wood, for fighting slime in paper and pulp industry, for fighting trichophytid fungi, generally for fighting fungal contaminations and/or preventing spoiling of foodstuffs, particularly for preventing spoiling of bakery products, for preventing spoiling of meat products, prepared foods and/or fast food, for preventing spoiling of jams, berry and fruit preserves and/or sweets and/or preventing spoiling of products of the dairy industry, said microbe inhibitor and/or microbe inhibitor fraction being made from barley in that the barley has been ground, the meal has been suspended in water for dissolving the soluble substances, the non-soluble constituents have been removed and from the solution has been separated a microbe inhibitor fraction having molecular weight 7000 to 30,000.

10. The use of a microbe inhibitor and/or microbe inhibitor fraction according to claim 9, the known inhibitor being nisin or some chemical inhibitor.

11. The use of a microbe inhibitor and/or microbe inhibitor fraction according to claim 9 or 10, the microbe inhibitors being used at a temperature lower than 50°C.

12. Food product, **characterized** in that it contains microbe inhibitor and/or microbe inhibitor fraction according to any of the claims 5 - 8.

13. Food product according to claim 12, **characterized** in that the product is bakery product, meat, meat product, prepared food products, fast food, jam, berry, sweets, dairy industry product, salad and dressing.

## Patentansprüche

1. Verfahren zum Abtrennen eines Mikrobeninhibitors und/oder einer Kombination von Mikrobeninhibitoren von Getreide, **dadurch gekennzeichnet,** daß Gerste gemahlen wird, das Mehl in Wasser suspendiert wird, um die löslichen Substanzen aufzulösen, die unlöslichen Bestandteile entfernt werden und von der Lösung eine Mikrobeninhibitorfraktion mit einem Molekulargewicht von 7000 bis 30000 abgetrennt wird.

2. Verfahren nach Anspruch 1, **dadurch gekennzeichnet,** daß von der Lösung eine Mikrobeninhibitorfraktion mit einem Molekulargewicht von 10000 bis 25000 abgetrennt wird.

3. Verfahren nach Anspruch 2, **dadurch gekennzeichnet,** daß die Inhibitorfraktion konzentriert wird, vorteilhafterweise durch Zentrifugieren, Ultrafiltration, Gelfiltration und/oder Eindampfen.

4. Verfahren nach einem der Ansprüche 1-3, **dadurch gekennzeichnet,** daß von der wäßrigen Suspension von Gerstenmehl die Stärkesubstanzen und Faserstoffe durch Seihen, Filtration, Zentrifugieren oder ein anderes bekanntes Trennverfahren abgetrennt werden und von der so erhaltenen Lösung die Mikrobeninhibitorfraktion durch Zentrifugieren, Ultrafiltration, Gelfiltration, Eindampfen oder ein anderes bekanntes Trennverfahren abgetrennt wird.

5. Mikrobeninhibitor und/oder Mikrobeninhibitorfraktion, welche(r) von Getreide abgetrennt worden ist, **dadurch gekennzeichnet,** daß Gerste gemahlen worden ist, das Mehl zum Auflösen der löslichen Substanzen in Wasser suspendiert worden ist, die nichtlöslichen Bestandteile entfernt worden sind und von der Lösung eine Mikrobeninhibitorfraktion mit einem Molekulargewicht von 7000 bis 30000 abgetrennt worden ist.

6. Mikrobeninhibitor und/oder Mikrobeninhibitorfraktion nach Anspruch 5, **dadurch gekennzeichnet,** daß von der Lösung eine Mikrobeninhibitorfraktion mit einem Molekulargewicht von 10000 bis 25000 abgetrennt worden ist.

7. Mikrobeninhibitor und/oder Mikrobeninhibitorfraktion nach Anspruch 5 oder 6, **dadurch gekennzeichnet,** daß die Mikrobeninhibitorfraktion konzentriert worden ist, vorteilhafterweise durch Zentrifugieren, Ultrafiltration, Gelfiltration und/oder Eindampfen.

8. Mikrobeninhibitor und/oder Mikrobeninhibitorfraktion nach einem der Ansprüche 5-7, **dadurch gekennzeichnet,** daß die Mikrobeninhibitorfraktion durch Abtrennen der Stärkesubstanzen und Faserstoffe von der wäßrigen Suspension von Gerstenmehl durch Seihen, Filtration, Zentrifugieren oder ein anderes bekanntes Trennverfahren hergestellt worden ist und von der so erhaltenen Lösung der Mikrobeninhibitor durch Zentrifugieren, Ultrafiltration, Gelfiltration, Eindampfen oder ein anderes bekanntes Trennverfahren abgetrennt worden ist.

9. Verwendung eines Mikrobeninhibitors und/oder einer Mikrobeninhibitorfraktion als solche und gegebenenfalls mit einem bekannten Inhibitor zum Verzögern oder Verhindern des Wachstums von Bakterien und/oder Pilzen, zum Schützen von Holz, zum Bekämpfen von Schleim in der Papier- und Zellstoffindustrie, zum Bekämpfen von trichophytiden Pilzen, allgemein zum Bekämpfen von Kontaminationen durch Pilze und/oder zum Verhindern des Verderbs von Lebensmitteln, insbesondere zum Verhindern des Verderbs von Backwaren, zum Verhindern des Verderbs von Fleischprodukten, Fertiggerichten und/oder Schnellgerichten, zum Verhindern des Verderbs von Marmeladen, Beeren- und Fruchtkonserven und/oder Konfekt und/oder zum Verhindern des Verderbs von Produkten der Milchindustrie, wobei der Mikrobeninhibitor und/oder die Mikrobeninhibitorfraktion aus Gerste hergestellt worden sind, indem die Gerste gemahlen worden ist, das Mehl zum Auflösen der löslichen Substanzen in Wasser suspendiert worden ist, die nichtlöslichen Bestandteile entfernt worden sind und von der Lösung eine Mikrobeninhibitorfraktion mit einem Molekulargewicht von 7000 bis 30000 abgetrennt worden ist.

10. Verwendung eines Mikrobeninhibitors und/oder einer Mikrobeninhibitorfraktion nach Anspruch 9, wobei der bekannte Inhibitor Nisin oder ein chemischer Inhibitor ist.

11. Verwendung eines Mikrobeninhibitors und/oder einer Mikrobeninhibitorfraktion nach Anspruch 9 oder 10, wobei die Mikrobeninhibitoren bei einer Temperatur unter 50°C verwendet werden.

12. Lebensmittelprodukt, **dadurch gekennzeichnet,** daß es einen Mikrobeninhibitor und/oder eine Mikrobeninhibitorfraktion nach einem der Ansprüche 5 - 8 enthält.

13. Lebensmittelprodukt nach Anspruch 12, **dadurch gekennzeichnet,** daß es sich bei dem Produkt um Backwaren, Fleisch, ein Fleischprodukt, Fertiggerichtprodukte, ein Schnellgericht, Marmelade, Beeren, Konfekt, ein Milchindustrieprodukt, Salat und Dressing handelt.

## Revendications

1. Procédure de séparation d'un inhibiteur de microbes et/ou d'une combinaison d'inhibiteurs de microbes d'une céréale caractérisée en ce que de l'orge est moulue, la farine est mise en suspension dans l'eau de manière à dissoudre les substances solubles, les constituants insolubles sont éliminés et l'on sépare de la solution une fraction d'inhibiteur de microbes présentant une masse moléculaire de 7 000 à 30 000.

2. Procédure selon la revendication 1, caractérisée en ce que l'on sépare de la solution une fraction d'inhibiteur de microbes présentant une masse moléculaire de 10 000 à 25 000.

3. Procédure selon la revendication 2, caractérisée en ce que la fraction d'inhibiteur est concentrée avantageusement à l'aide d'une centrifugation, d'une ultrafiltration, d'une filtration sur gel et/ou d'une évaporation.

4. Procédure selon l'une quelconque des revendications 1 à 3, caractérisée en ce que l'on sépare de-la suspension aqueuse de farine d'orge, les substances amidonnées et les substances fibreuses à l'aide d'un égouttage, d'une filtration, d'une centrifugation ou autre procédé de séparation connu et que l'on sépare, de la solution ainsi obtenue, la fraction d'inhibiteur de microbes par centrifugation, ultrafiltration, filtration sur gel, évaporation ou un autre procédé de séparation connu.

5. Inhibiteur de microbes et/ou fraction d'inhibiteur de microbes qui ont été séparés d'une céréale, caractérisé en ce que de l'orge a été moulue, la farine a été mise en suspension dans l'eau en vue de dissoudre les substances solubles, les constituants non solubles ont été éliminés et l'on a séparé de la solution une fraction d'inhibiteur de microbes présentant une masse moléculaire de 7 000 à 30 000.

6. Inhibiteur de microbes et/ou fraction d'inhibiteur de microbes selon la revendication 5, caractérisé en ce que l'on a séparé de la solution une fraction d'inhibiteur de microbes présentant une masse moléculaire de 10 000 à 25 000.

7. Inhibiteur de microbes et/ou fraction d'inhibiteur de microbes selon la revendication 5 ou 6, caractérisé en ce que la fraction d'inhibiteur de microbes a été concentrée avantageusement à l'aide d'une centrifugation, d'une ultrafiltration, d'une filtration sur gel et/ou d'une évaporation.

8. Inhibiteur de microbes et/ou fraction d'inhibiteur de microbes selon l'une quelconque des revendications 5 à 7, caractérisé en ce que la fraction d'inhibiteur de microbes a été préparée en séparant de la suspension aqueuse de farine d'orge, les substances amidonnées et les substances fibreuses à l'aide d'un égouttage, d'une filtration, d'une centrifugation ou autre procédé de séparation connu et que l'inhibiteur de microbes a été séparé de la solution ainsi obtenue par centrifugation, ultrafiltration, filtration sur gel, évaporation ou un aucre procédé de séparation connu.

9. Utilisation d'un inhibiteur de microbes et/ou d'une fraction d'inhibiteur de microbes, en tant que tel et éventuellement avec un inhibiteur connu quelconque en vue de retarder ou d'empêcher la croissance de bactéries et/ou de champignons, en vue de protéger le bois, en vue de combattre la vase dans l'industrie du papier et de la pâte à papier, afin de combattre les champignons trichophytides, d'une manière générale afin de combattre les contaminations par les champignons et/ou empêcher l'altération des produits alimentaires, en particulier en vue d'empêcher l'altération des produits de boulangerie, en vue d'empêcher l'altération des produits de boucherie, des aliments préparés et/ou de l'alimentation rapide et, en vue d'empêcher l'altération des confitures, des conserves de baies et de fruits et/ou des sucreries et/ou empêcher l'altération des produits de l'industrie laitière, ledit inhibiteur de microbes et/ou fraction d'inhibiteur de microbes étant obtenu à partir d'orge, en ce que l'orge a été moulue, la farine a été mise en suspension dans l'eau en vue de dissoudre les substances solubles, les constituants non solubles ont été éliminés et l'on a séparé de la solution une fraction d'inhibiteur de microbes présentant une masse moléculaire de 7 000 à 30 000.

10. Utilisation d'un inhibiteur de microbes et/ou d'une fraction d'inhibiteur de microbes selon la revendication 9, l'inhibiteur connu étant la nisine ou un certain inhibiteur chimique.

11. Utilisation d'un inhibiteur de microbes et/ou d'une fraction d'inhibiteur de microbes selon la revendication 9 ou 10, les inhibiteurs de microbes étant utilisés à une température inférieure à 50°C.

12. Produit alimentaire, caractérisé en ce qu'il contient un inhibiteur de microbes et/ou une fraction d'inhibiteur de microbes selon l'une quelconque des revendications 5 à 8.

13. Produit alimentaire selon la revendication 12, caractérisé en ce que le produit est un produit de boulangerie, de la viande, un produit de boucherie, des produits alimentaires préparés, une alimentation rapide, de la confiture, des baies, des sucreries, un produit d'industrie laitière, de la salade et un assaisonnement.
